# EUROPEAN PATENT APPLICATION

(11) **EP 4 593 028 A1**
(43) Date of publication of application: **30.07.2025**
(21) Application number: 23894845.9
(22) Date of filing: 27.10.2023
(51) Int. Cl.: G16H 30/40, G16H 30/20, G06V 10/82, G06V 10/75, A61B 5/055, A61B 6/03, G06N 3/08

(54) **METHOD FOR MEDICAL IMAGE CONVERSION IN FREQUENCY DOMAIN USING ARTIFICIAL INTELLIGENCE, AND DEVICE THEREOF**

(30) Priority: 24.11.2022 KR 20220158815
(71) Applicant: Polestar Healthcare Co., Ltd., Seoul 06627 (KR)
(72) Inventor: YOON, Yeo Dong, Seoul 06629 (KR); LEE, Eun Chong, Yongin-si Gyeonggi-do 16812 (KR)
(74) Representative: RGTH
(86) International application number: PCT/KR2023/016878
(87) International publication number: WO 2024/111916

(57) **Abstract**

One mode of the present invention relates to a medical image conversion method, and, more specifically, to a medical image conversion method using a generative adversarial network (GAN). An embodiment of the present invention has the effect of providing a medical image conversion method that enables a medical team to perform medical examination, diagnosis, and treatment using accurate information through conversion between medical images on the basis of a machine learning model, and enables a patient to receive suitable medical services.

## Description

### TECHNICAL FIELD

An aspect of the disclosure relates to a method and device for converting a medical image and, more particularly, to an image conversion method and device for converting a medical image in a frequency domain using a generative adversarial network (GAN).

### BACKGROUND ART

The information disclosed in this section is only provided for an understanding of background information of embodiments of the disclosure and should not be taken as a description of the prior art.

For the diagnosis and treatment of emergency room patients with brain hemorrhage, tumor patients, and the like, non-enhanced (or non-contrast enhanced) CT images in which no contrast enhancers are used, and enhanced (or contrast enhanced) CT images in which contrast enhancers are used may be widely used.

For example, contrast agents for enhancing tissue contrast may be useful to detect blood vessels, organs, cancer, or the like. However, contrast agents may often cause side effects and even death due to cardiac arrest, shock death, or the like.

Because such side effects may not be predicted in advance, image conversion of non-enhanced CT or MR images to enhanced CT or MR images may help prevent these side effects and reduce the medical costs of contrast agents.

In addition, for individuals such as pregnant women who have MR images but no CT images due to unavailability of X-ray radiation or individuals who have CT images but are not MR imaged for reasons such as lack of time, cost, or implants, image conversion between imaging modalities may also help medical professionals to identify tissue boundaries or locate tumors without the use of contrast agents.

Furthermore, conversion between MR images, such as from a T1 image to a T2 image or from a T1 image to a diffusion image, may help medical professionals better understand lesions.

In addition, according to learning methods of general learning models, a learning model receives an input image, synthesizes an output image based on the input image, compares the synthesized output image with a target image, and reduces the difference between the output image and the target image.

In these learning methods of general learning models, the output image is constructed in an image domain. In a case in which an image in a frequency domain of the output image and an image in a frequency domain of target image have the same frequency waveforms, such as the same high-frequency components, the output image may be produced more clearly. Therefore, it is worth studying Al learning models in the frequency domain.

The information disclosed in the Background section is technical information that the inventors possessed for, or acquired during, derivation of embodiments of the disclosure and should not be taken as known technology disclosed to the public before the filing of the embodiments of the disclosure.

### DISCLOSURE

### Technical Problem

Accordingly, an aspect of the disclosure has been made to solve the above-described problems, and an objective of the disclosure is to provide a method and device for converting a medical image by a learning model which performs training of converting both an output image and a target image into frequency domain images (i.e., images in a frequency domain) and comparing the converted images in the frequency domain to reduce the difference of the images, thereby obtaining a clear output image.

Another objective of the disclosure is to provide a method of converting a medical image which enables medical professionals to perform medical examination, diagnosis, and treatment with accurate information and patients to receive appropriate medical services.

The objectives of the disclosure are not limited to the foregoing description, and other objectives not explicitly disclosed herein will be clearly understood by a person having ordinary knowledge in the art to which the disclosure pertains from the description provided hereinafter.

### Technical Solution

In order to achieve at least one of the above objectives, an aspect of the disclosure provides a method of converting a medical image using a generative adversarial network (GAN), the method including:
a first operation of receiving a first image selected from a paired data set including the first image and a second image;
a second operation of constructing a third image matching the first image based on the first image; and a third operation of converting the second image and the third image to frequency domain images, respectively, comparing the converted frequency domain images, and reflecting the comparison result to a learning model performing the conversion of medical images.

In some embodiments, in the third operation, the frequency domain image may include an image in K-space.

In some embodiments, the third operation may include: comparing a second frequency domain image to which the second image is converted in the frequency domain and a third frequency domain image to which the third image is converted in the frequency domain and reflecting a comparison result to the learning model (which trains in the frequency domain) to derive a quality-enhanced third frequency domain image; and

inverse-converting the quality-enhanced third frequency domain image to an image in the image domain.

Another aspect of the disclosure provides a device for converting a medical image using a generative adversarial network (GAN), the device including:
a first learning model receiving a first image selected from a paired data set including the first image and a second image and constructing a third image based on the first image; and
a second learning model receiving a third frequency domain image to which the third image is converted in the frequency domain and constructing a quality-enhanced third frequency domain image based on the third frequency domain image.

In some embodiments, the device may include: a comparator comparing a second frequency domain image to which a second image is converted in the frequency domain with a third frequency domain image to which the third image is converted in the frequency domain and feeding back the difference to the second learning model.

In some embodiments, the device may include: an inverse-conversion part inversely converting the quality-enhanced third frequency domain image to construct a quality-enhanced third image.

In some embodiments, the third frequency domain image, the second frequency domain image, and the quality-enhanced third frequency domain image may be images in K-space.

Another aspect of the disclosure provides a method of converting a medical image using a generative adversarial network (GAN), the method including:
an image construction operation of receiving a first image and constructing a third image;
a first frequency conversion operation of converting the third image to a third frequency domain image in the frequency domain; and
an image quality enhancement operation of receiving the third frequency domain image and constructing a quality-enhanced third frequency domain image.

In some embodiments, the method may include: a second frequency conversion operation of converting a second image constituting a paired data set with the first image to a second frequency domain image in the frequency domain; and
an image comparison operation of comparing the second frequency domain image and the third frequency domain image and reflecting the difference to the image quality enhancement operation.

In some embodiments, the method may include an inverse-conversion operation of inversely converting the quality-enhanced third frequency domain image and outputting a quality-enhanced third image.

### Advantageous Effects

As set forth above, an embodiment of the disclosure provides a method and device for converting a medical image by a learning model which performs training of converting both an output image and a target image into frequency domain images (i.e., images in a frequency domain) and comparing the converted images in the frequency domain to reduce the difference of the images, thereby obtaining a clear output image.

Another embodiment the disclosure provides a method of converting a medical image which enables medical professionals to perform medical examination, diagnosis, and treatment with accurate information and patients to receive appropriate medical services.

In addition, the effects of the disclosure have various effects, including excellent generalizability, for example, depending on the embodiments, which will become apparent from the following description of the embodiments.

### DESCRIPTION OF DRAWINGS

The following drawings accompanying the specification illustrate embodiments of the disclosure and, together with the foregoing disclosure, serve to provide further understanding of the technical spirit of the disclosure, and thus, the disclosure should not be construed as being limited to the drawings, wherein:
FIG. 1 illustrates a method of converting a medical image according to an embodiment of the disclosure.
FIG. 2 illustrates an Al learning model according to an embodiment of the disclosure.
FIG. 3 illustrates an embodiment of calculating the match rate between an output image and a target image.
FIG. 4 illustrates a method of converting a medical image according to another embodiment of the disclosure.
FIG. 5 illustrates a method of converting a medical image according to another embodiment of the disclosure.
FIG. 6 illustrates an Al learning model according to another embodiment of the disclosure.
FIGS. 7 and 8 illustrate a method of converting a medical image according to another embodiment of the disclosure.

### BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the spirit and scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

The terminology used herein is for the purpose of describing particular embodiments only and is not intended to be limiting. As used herein, the singular forms are intended to include the plural forms as well, unless the context clearly indicates otherwise.

Terms, such as "comprise/include" or "have", or the like, as used herein, indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar components are given the same reference numerals, and repeated descriptions thereof will be omitted.

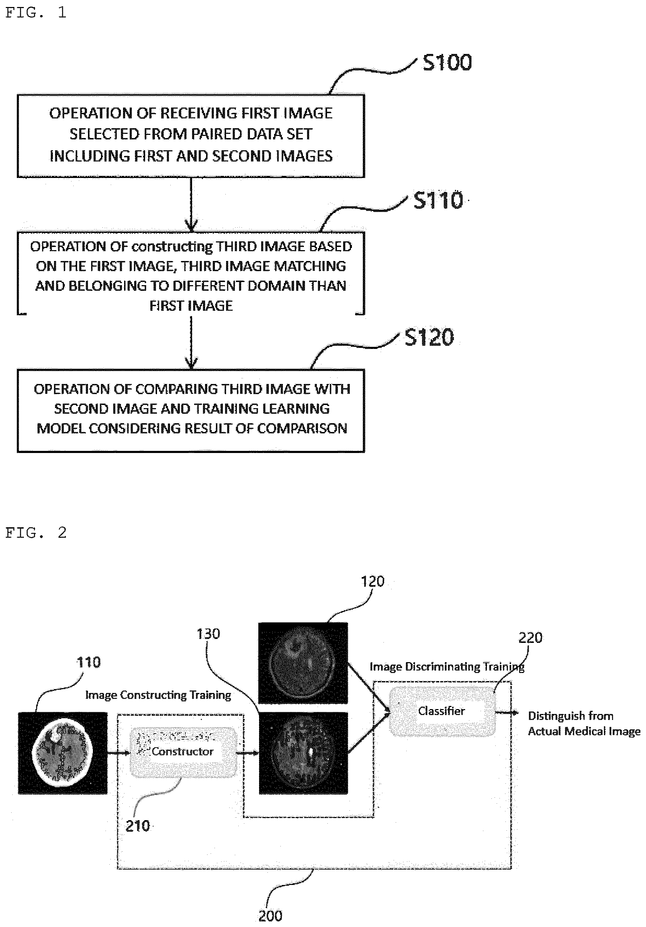

FIG. 1 illustrates a method of converting a medical image according to an embodiment of the disclosure, and FIG. 2 illustrates an artificial intelligence (AI) learning model according to an embodiment of the disclosure.

According to an embodiment of the disclosure, a method of converting a medical image using a generative adversarial network (GAN) may be provided.

The method of converting a medical image according to this embodiment may include: an input operation S100 of receiving a first image 110 selected from a paired data set including the first image 110 and a second image 120; a construction operation S110 of constructing a third image 130 based on the first image 110, the third image 130 matching the first image 110 and belonging to a different domain from the first image 110;

a training operation S120 of comparing the third image 130 with the second image 120 and training the learning model considering a result of the comparison.

The first image 110 and the second image 120 may be medical images. The medical images may be magnetic resonance images, such as 2D MR, 3D MR, 2D streaming MR, 4D MR, 4D volumetric MR, and 4D cine MR images; functional MR images, such as fMR, DCE-MR, and diffusion MR images; computed tomography (CT) images, such as 2D CT, cone beam CT, 3D CT, and 4D CT images; ultrasound images, such as 2D ultrasound, 3D ultrasound, and 4D ultrasound images; positron emission tomography (PET) images; X-ray images; fluoroscopic images; radiotherapy portal images; single-photon emission computed tomography (SPECT) images; computer generated synthetic images, such as pseudo-CT images; and the like.

Furthermore, the medical images may include medical image data, such as a training image, a ground truth image, a contoured image, a dose image, and the like.

In some embodiments, the medical image may include an image acquired using an image acquisition device, a computer generated synthetic image, and the like. The image acquisition device may include, for example, an MR imaging device, a CT imaging device, a PET imaging device, an ultrasound imaging device, a fluoroscopic device, a SPECT imaging device, an integrated linear accelerator, an MR imaging device, and the like. Furthermore, the image acquisition device is not limited to the above-described examples and may include a variety of medical imaging devices within the scope of the technical idea for acquiring medical images of a patient.

A paired data set may include a set of training data including images of an object such as a particular part of a patient.

A CT imaging device and an MR imaging device may be used to acquire a CT image and an MR image, respectively, of a particular part of a patient, such as the head, in which case the CT image and the MR image may form a paired data set.

In a case in which a non-enhanced (or non-contrast enhanced) CT image and an MR image are respectively acquired for a particular part of a patient, the non-enhanced CT image and the MR image may form a paired data set. In a case in which an enhanced (or contrast enhanced) CT image and an MR image are respectively acquired, the enhanced CT image and the MR image may form a paired data set.

In a case in which T1 and T2 images are acquired from a particular part of a patient, the T1 and T2 images may form a paired data set.

In this context, the particular part of a patient may mean the particular part of a single patient. In a case in which imaged parts are the same, the imaged parts may refer to particular parts of different patients.

For example, CT and MR images acquired from a particular part of a single patient may form a paired data set, and CT and MR images acquired from the same parts of different patients may also form a paired data set.

Different domains of two images mean that the domains are different, for example, in a case in which respective patterns contained in two matching images have different contrasts or two images have different intensity distributions or different patterns.

Furthermore, images acquired using different imaging devices belong to different domains. For example, because CT and MR images are acquired using a CT imaging device and an MR imaging device, respectively, both images belong to different domains. Similarly, there are domain differences between ultrasound and CT images, between ultrasound and MR images, and between MR and PET images.

Furthermore, non-enhanced and enhanced images have different intensity distributions for the same part, and thus belong to different domains.

For MR images, T1 and T2 images are the same MR images, but have different intensities and therefore belong to different domains. That is, in a case in which two images realized by reconstruction based on signals provided from an object have a difference in signals, sequences, or image realization processes on which the image realization is based, the two images having the difference belong to different domains. Furthermore, the medical image examples described above belong to different domains.

To illustrate the method of converting a medical image according to an embodiment of the disclosure, a case in which the image acquisition device has obtained a paired data set by acquiring a first image 110 and a second image 120 from the same part of a single patient will be described as an example. A case in which the first image 110 is an original CT image, the second image 120 is an original MR image, and the third image 130 is a synthetic MR image according to embodiments will be described.

The input operation S100 may include receiving, by the learning model, the first image 110 selected from the paired data set including the first image 110 and the second image 120. The first image 110 may include an input image processed from an original CT image with training data.

In the paired data set including the first image 110 and the second image 120, which one of the first image 110 and the second image 120 is to be selected as the input image may be determined by the manufacturer of medical device software which performs the method of converting a medical image according to this embodiment. In this case, which image is to be selected may be selected automatically by a selection algorithm, or may be selected manually by an operator.

The manufacturer may acquire the original CT image from the CT imaging device and the original MR image from the MR imaging device to form a paired data set, and may select the original CT image as the input image from the paired data set by an algorithm or manually.

The construction operation S100 may include constructing the third image 130 based on the first image 110, in which the learning model having received the first image 110 constructs the third image 130 which matches the first image 110 and belongs to a different domain from the first image 110.

The learning model receives the original CT image as an input image and constructs a synthetic MR image based on the original CT image.

The process of the learning model constructing the third image 130 from the first image 110 which belongs to a different domain from the first image 110 may be performed by the following two processes according to embodiments. However, the process of the learning model constructing the third image 130 is not limited to the following two processes.

The first process may include one or more layers which receive the first image 110 obtained from the image acquisition device and perform convolutional operations to extract high-dimensional features having a smaller size than the first image 110, in which the layers may be represented by an artificial neural network configured to extract features. Such a feature extraction artificial neural network may receive two- or three-dimensional real image data obtained from the image acquisition device and perform convolutional operations to extract image features from the original images.

Describing this process by way of example, a convolutional layer which extracts the features of an image through a filter and a pooling layer which enhances the features and reduces the size of the image may be provided to extract the features of an image by repeating the convolution and the pooling.

In some embodiments, a convolutional layer including a plurality of layers may extract features of an input image given as an input from a first convolutional layer and produce a feature map as a result, a pooling layer may receive this feature map and produce a result having enhanced features and a reduced image size, and the output of the pooling layer may be input again into a second convolutional layer.

The second process includes one or more layers performing a deconvolution operation for the purpose of generating image data of different modalities from the output of the first process described above, in which these layers may be represented by an artificial neural network for generating images of different modalities. The artificial neural network which generates images of different modalities may perform a deconvolution operation on the result of the convolution operation of extracting the features by the artificial neural network in the first process to generate two- or three-dimensional image data of different modalities.

The training operation S120 may include operations of comparing the third image 130 with the second image 120 and training the learning model considering the comparison result. In a case in which the original CT image in the paired data set which includes the original CT image and the original MR image is determined to be the input image, the original MR image may be determined to be the target image. The second image 120 may correspond to ground truth. After constructing the third image 130, the learning model may be trained to reduce the difference between the constructed third image 130 and the second image 120 by comparing the constructed third image 130 with the second image 120. That is, the learning model may be trained to compare the synthetic MR image with the original MR image and continuously review whether the synthetic MR image is constructed to be similar to the original MR image, so that the synthetic MR image and the original MR image are the same. In some embodiments, an operation of calculating a loss value between the third image 130 and the second image 120 by applying a loss function to the algorithm may be included, and the parameters of the learning model may be updated based on the loss value.

In some embodiments, in the operation of calculating a loss value, the calculation of the loss value to update the learning model may be performed at each iteration. That is, the learning model may calculate the loss value between the synthetic MR image and the original MR image at each iteration during the training.

In a case in which the iteration includes a first iteration section and a second iteration section which are sequential, in some embodiments, the learning model may recognize overfitting and terminate the training in a case in which the absolute value of the change between the first loss value calculated in the first iteration section and the second loss value calculated in the second iteration section is less than a reference value.

In a case in which the learning model is sufficiently trained for the medical image conversion, the training may no longer be performed and be completed. The model having completed the training is now able to convert medical images into third images 130 with respect to a plurality of first images 110 having different data distributions, the third images 130 belonging to different domains from the first images 110.

A learning model 200 according to an embodiment of the disclosure may use a GAN model, and may include a constructor and a discriminator. The constructor may be trained to construct images, and the discriminator may be trained to discriminate images.

Referring to FIG. 2, in the learning model 200 using the GAN model, in a case in which the original CT image 110 is input as an input image to a constructor 210, the constructor 210 may be trained to construct a synthetic MR image 130 by the convolutional operation described above, and a discriminator 220 may be trained to discriminate the synthetic MR image 130 from an original MR image 120, i.e., a real medical image.

In some embodiments, the learning model 200 according to this embodiment may use a cycle GAN model (not shown). In this case, the learning model 200 may include the first constructor 210, the first discriminator 220, a second constructor, and a second discriminator. In a case in which the original CT image 110 is input to the first constructor 210 as an input image, the first constructor 210 may be trained to construct the synthetic MR image 130 through the convolutional operation described above, and the first discriminator 220 may be trained to discriminate a synthetic MR image from the original MR image 120, i.e., the real medical image. In a case in which the synthetic MR image 130 constructed by the first constructor 210 is then input to the second constructor, the second constructor may be trained to construct a synthetic CT image based on the synthetic MR image 130, and the second discriminator may be trained to discriminate a synthetic CT image from an original CT image.

Here, the original CT image 110 may correspond to the first image 110 in the method of converting a medical image described above, the synthetic MR image 130 may correspond to the third image 130, and the original MR image 120 may correspond to the second image 120.

In some embodiments, the training operation S120 may include calculating a match rate between the third image 130 and the second image 120 and feeding the calculated value back to the learning model. Here, calculating the match rate is a method for determining the degree of difference between two images belonging to the same domain.

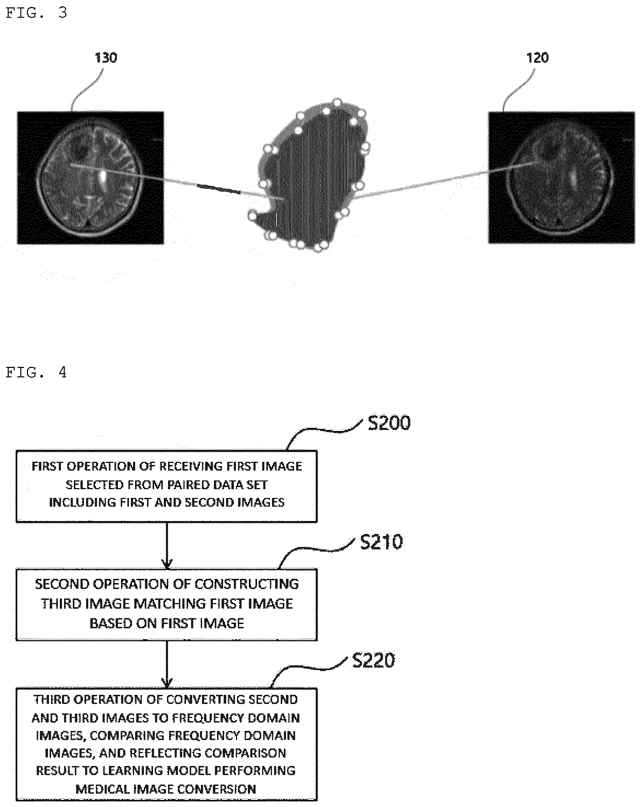

FIG. 3 illustrates an embodiment of calculating the match rate between an output image and a target image.

In some embodiments, in a case in which a lesion region is marked on each of the same parts of a synthetic MR image and an original MR image, the calculation of the match rate may be performed by comparing the sizes of the lesion regions. For example, the match rate may be calculated by marking the location of a tumor or other lesion in the synthetic MR image and the original MR image, respectively, followed by conversion to a binary image.

In this case, the match rate may be calculated using an evaluation metric, such as the Hausdorff distance and the Dice similarity coefficient, or calculated quantitatively based on the difference between the centers of mass of the two lesion locations. Furthermore, in a case in which the target image and the output image are CT images, the match rate of the two images may be determined by comparing the radiation dose calculations for the lesions. In some embodiments, the determination may be performed by representing the difference between the synthetic MR image and the original MR image using numerical values such as MAE, RMSE, SSIM, or PSNR (hereinafter referred to as a performance metric).

Referring to FIG. 3, the process of calculating the match rate by aligning the lesion regions of the synthetic MR image 130, i.e., the output image, and the original MR image 120, i.e., the target image, and comparing the contours of the respective lesions using the Hausdorff distance measure is illustrated.

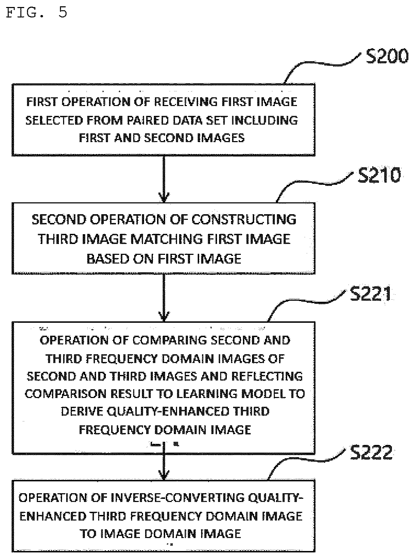

FIG. 4 illustrates a method of converting a medical image according to another embodiment of the disclosure, and FIG. 5 illustrates a method of converting a medical image according to another embodiment of the disclosure.

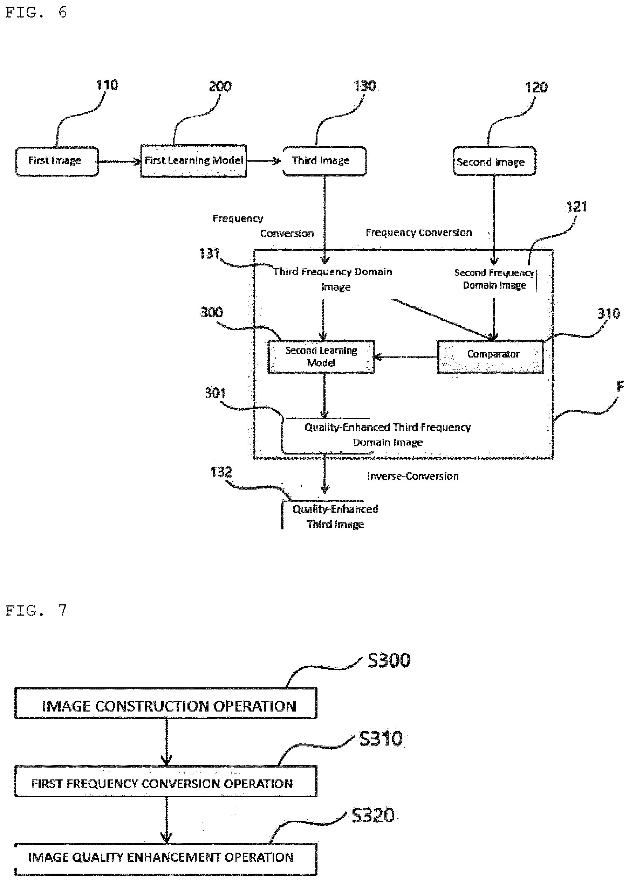

FIG. 6 illustrates an Al learning model according to another embodiment of the disclosure.

A method of converting a medical image according to an embodiment of the disclosure is used for the conversion of a medical image using a generative adversarial network (GAN).

The method of converting a medical image according to this embodiment may include: a first operation S200 of receiving a first image 110 selected from a paired data set including the first image 110 and a second image;
a second operation S210 of constructing a third image matching the first image 110 based on the first image 110; and
a third operation S220 of converting the second image and the third image to frequency domain images (i.e., images in a frequency domain), respectively, comparing the converted frequency domain images, and reflecting the comparison result to a learning model 300 which performs medical image conversion. The learning model 300 referred to herein may mean a learning model which performs training in the frequency domain. This learning model will be referred to as a second learning model 300 in embodiments described later.

In this embodiment, the first image 110 may refer to an input image, which is training data input to the learning model, the second image 120 may refer to a target image, and the third image 130 may refer to an output image.

In some embodiments, the frequency domain image in the third operation S220 may include an image in a K-space.

As used herein, the K-space may correspond to an image domain.

In the K-space, a signal having multiple position information may be obtained by applying an RF pulse, for example, in MR imaging and then changing the magnitude of a phase encoding gradient (G) for respective operations. Such data is referred to as raw data. The raw data has both position and contrast information, and the K-space may refer to a set of raw data that may form a single image.

The K-space contains all information about the image in the frequency domain. That is, distortion and loss of particular portions of the K-space will inevitably result in changes in the image.

For example, when a K-space in which high-frequency components are lost is compared with the corresponding image domain, it may be seen that as the high-frequency components corresponding to the edges in the K-space are gradually lost, the image in the image domain becomes slightly blurry. This is due to the breakdown of the boundaries within the image corresponding to the high-frequency components.

Furthermore, when a K-space in which the low-frequency components are lost is compared with the corresponding image domain, it may be seen that as the low-frequency components corresponding to the center portion of the K-space are gradually lost, only the borderline portion of the image, which is the high-frequency component, is left, and the brightness and contrast are destroyed.

In the disclosure, the K-space may contain all the information in the image domain. In other words, depending on how the information in the K-space is utilized, a designer may get the desired shape of the image. For example, if a designer wants an image with only edge components, the designer may blow out the low-frequency components, or if a designer wants a smoothing effect, the designer may lose the high-frequency components.

In some embodiments, the third operation S220 may include: an operation S221 of comparing a second frequency domain image 121 to which the second image is converted in the frequency domain and a third frequency domain image 131 to which the third image is converted in the frequency domain and reflecting a comparison result to the learning model to derive a quality-enhanced third frequency domain image 301; and an operation S222 of inverse-converting "the quality-enhanced third frequency domain image" 301 to an image in the image domain.

Finally, an "quality-enhanced third image" 132 may be output by inverse-converting the "quality-enhanced third frequency domain image" 301 to an image in the image domain. Although the "quality-enhanced third image" is an image in the image domain such as the third image 130, the quality-enhanced third image is an output image output by the learning model 300 performing training in the frequency domain, so that the quality-enhanced third image may show enhanced results over the output image output by the learning model 200 which simply performs training in the image domain. In this context, the quality enhancement may refer to an increase in image sharpness, for example.

By comparing the third frequency domain image 131 and the second frequency domain image, which are images in the frequency domain, and feeding back the calculated value to the learning model 300 that trains in the frequency domain so that the learning model 300 may match the third frequency domain image and the second frequency domain image, the third image 130 and the second image 120 may match in the image domain. In other words, the learning model 300 may match the frequency shape in the frequency domain to more precisely match the images in the image domain.

Another aspect of the disclosure provides a medical image conversion device using a generative adversarial network (GAN).

The device may include: a first learning model 200 receiving a first image 110 selected from a paired data set including the first image 110 and a second image and constructing a third image based on the first image 110; and
a second learning model 300 receiving a third frequency domain image 131 to which the third image is converted in the frequency domain and constructing a quality-enhanced third frequency domain image 301 based on the third frequency domain image 131. Here, the first learning model 200 may include a model that trains in the image domain, and the second learning model 300 may include a model that trains in the frequency domain.

The medical image conversion device of this embodiment may further include a comparator 310 comparing a second frequency domain image 121 to which a second image is converted in the frequency domain with a third frequency domain image 131 to which the third image is converted in the frequency domain and feeding back the difference to the second learning model 300.

In some embodiments, the comparator 310 serves to compare the frequency shapes of the second frequency domain image 121 and the third frequency domain image 131 in the frequency domain.

In some embodiments, the medical image conversion device may further include an inverse-conversion part inversely converting the quality-enhanced third frequency domain image 301 to construct a quality-enhanced third image 132. Here, the image quality-enhanced third image 132 may include an image having an increase in image sharpness.

In some embodiments, the third frequency domain image 131, the second frequency domain image 121, and the quality-enhanced third frequency domain image 301 may be images in K-space.

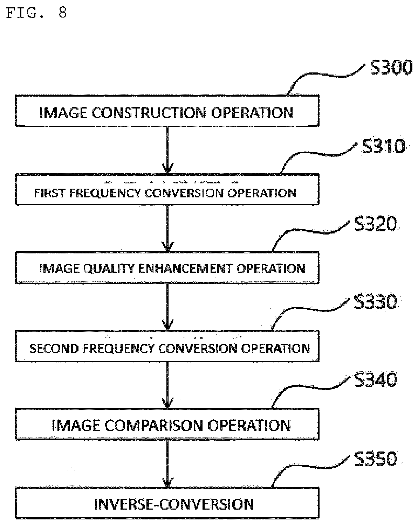

FIGS. 7 and 8 illustrate a method of converting a medical image according to another embodiment of the disclosure.

Another aspect of the disclosure provides a method of converting a medical image using a generative adversarial network (GAN).

The method may include: an image construction operation S300 of receiving a first image 110 and constructing a third image 130;
a first frequency conversion operation S310 of converting the third image to a third frequency domain image 131 in the frequency domain; and
an image quality enhancement operation S320 of receiving the third frequency domain image 131 and constructing a quality-enhanced third frequency domain image 301.

Here, the image quality enhancement operation S320 may be performed by a learning model 300 that trains in the frequency domain. In other words, the learning model 300 may receive the third frequency domain image 131 as an input and construct the quality-enhanced third frequency domain image 301 as an output.

In some embodiments, the method may include: a second frequency conversion operation S330 of converting a second image constituting a paired data set with the first image 110 to a second frequency domain image 121 in the frequency domain; and
an image comparison operation S340 of comparing the second frequency domain image 121 and the third frequency domain image 131 and reflecting the difference to the image quality enhancement operation.

In some embodiments, the method may include an inverse-conversion operation of inversely converting the quality-enhanced third frequency domain image and outputting "a quality-enhanced third image". Here, the "quality-enhanced third image" 132 may refer to an image in the image domain that has increased image quality.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware devices such as ROMs, RAMs, and flash memories specifically configured to store program instructions and execute the program instructions.

Furthermore, the computer programs may be specifically designed and configured for the disclosure or may be known and available to a person having ordinary knowledge in the art of computer software. Examples of computer programs may include machine code produced by compilers and high-level language code executable on computers using interpreters.

In the specification of the disclosure, the use of the term "the" and similar denoting terms may correspond to both singular and plural forms. Furthermore, recitation of ranges of values herein are intended merely to refer to respective separate values falling within the respective ranges and, unless otherwise indicated herein, the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the spirit of the disclosure shall not be limited to the above-described embodiments, and the entire scope of the appended claims and equivalents thereof will fall within the scope and spirit of the disclosure.

### <Description of Reference Numerals of Drawings>

- 100:: Al learning model
- 110:: first image
- 120:: second image
- 121:: second frequency domain image
- 130:: third image
- 131:: third frequency domain image
- 132:: quality-enhanced third image
- 200:: first learning model
- 300:: second learning model
- 301:: quality-enhanced third frequency domain image
- 310:: comparator

## Claims

1. A method of converting a medical image using a generative adversarial network (GA), the method comprising:
a first operation of receiving a first image selected from a paired data set including the first image and a second image;
a second operation of constructing a third image matching the first image based on the first image; and
a third operation of converting the second image and the third image to frequency domain images, respectively, comparing the converted frequency domain images, and reflecting a result of the comparison to a learning model which performs medical image conversion.

2. The method of claim 1, wherein in the third operation, the frequency domain image comprises an image in a K-space.

3. The method of claim 1, wherein the third operation comprises:
comparing a second frequency domain image to which the second image is converted in a frequency domain and a third frequency domain image to which the third image is converted in the frequency domain and reflecting a result of the comparison to the learning model (which trains in the frequency domain) to derive a quality-enhanced third frequency domain image; and
inverse-converting the quality-enhanced third frequency domain image to an image in the image domain.

4. A device for converting a medical image using a generative adversarial network (GAN), the device comprising:
a first learning model configured to receive a first image selected from a paired data set including the first image and a second image and constructing a third image based on the first image; and
a second learning model receiving a third frequency domain image to which the third image is converted in a frequency domain and constructing a quality-enhanced third frequency domain image based on the third frequency domain image.

5. The device of claim 4, comprising:
a comparator comparing a second frequency domain image to which a second image is converted in the frequency domain with a third frequency domain image to which the third image is converted in the frequency domain and feeding the difference back to the second learning model.

6. The device of claim 5, comprising:
an inverse-conversion part inversely converting the quality-enhanced third frequency domain image to construct a quality-enhanced third image.

7. The device of claim 4, wherein the third frequency domain image, the second frequency domain image, and the quality-enhanced third frequency domain image are images in a K-space.

8. A method of converting a medical image using a generative adversarial network (GAN), the method comprising:
an image construction operation of receiving a first image and constructing a third image;
a first frequency conversion operation of converting the third image to a third frequency domain image in a frequency domain; and
an image quality enhancement operation of receiving the third frequency domain image and constructing a quality-enhanced third frequency domain image.

9. The method of claim 8, comprising:
a second frequency conversion operation of converting a second image constituting a paired data set with the first image to a second frequency domain image in the frequency domain; and
an image comparison operation of comparing the second frequency domain image and the third frequency domain image and reflecting the difference to the image quality enhancement operation.

10. The method of claim 9, comprising:
an inverse-conversion operation of inversely converting the quality-enhanced third frequency domain image and outputting a quality-enhanced third image.
